# FASCICULE DE BREVET EUROPEEN

(11) **EP 1 335 791 B1**
(45) Date de publication et mention de la délivrance du brevet: **06.10.2010**
(21) Numéro de dépôt: 01997345.2
(22) Date de dépôt: 21.11.2001
(51) Int. Cl.: B01F 17/14, B01F 17/16, B01F 3/08, A61Q 1/04, A61Q 1/02, A61Q 5/10, A61Q 19/00

(54) **COMPOSITION COSMETIQUE ET/OU DERMATOLOGIQUE STABILISEE CONTENANT UN COLORANT ET UN EMULSIONNANT**
EINEN FARBSTOFF UND EINEN EMULGATOR ENTHALTENDE, STABILISIERTE, KOSMETISCHE UND/ODER DERMATOLOGISCHE ZUSAMMENSETZUNG
STABILISED COSMETIC AND/OR DERMATOLOGICAL COMPOSITION CONTAINING A COLOURING AGENT AND AN EMULSIFIER

(30) Priorité: 21.11.2000 FR 0015018
(43) Date de publication de la demande: 20.08.2003
(73) Titulaire: Laboratoire Nuxe, 75010 Paris (FR)
(72) Inventeur: LECLERE, Jacques, F-45500 SAINT-GONDON (FR)
(74) Mandataire: L'Helgoualch, Jean
(86) Numéro de dépôt international: PCT/FR2001/003670
(87) Numéro de publication internationale: WO 2002/041983

(56) Documents cités:
- EP-A- 0 043 327
- EP-A- 0 200 839
- EP-A- 0 312 343
- EP-A- 0 706 788
- WO-A-91/02525
- WO-A-91/07945
- WO-A-92/05761
- WO-A-95/11000
- WO-A-98/07406
- WO-A-98/44896
- FR-A- 2 315 991
- FR-A- 2 408 387

## Description

La présente invention concerne une nouvelle composition cosmétique et/ou dermatologique contenant une association stabilisée d'un colorant et d'un émulsionnant, et plus particulièrement une nouvelle composition sous forme d'émulsion comprenant au moins un colorant liposoluble ou lipodispersible et présentant une excellente stabilité, ainsi qu'un procédé pour sa préparation.

Dans la préparation de compositions cosmétiques et dermatologiques, et tout particulièrement dans les compositions pour la teinture des cheveux et pour le maquillage, on utilise couramment des colorants qui peuvent être d'origine naturelle, végétale ou animale, ou obtenus par voie de synthèse minérale ou organique. Ainsi on a longtemps utilisé des pigments dans les compositions destinées au maquillage, et par exemple des pigments naturels ou des pigments obtenus par synthèse, par exemple des pigments à base d'oxyde de fer tels que l'oxyde ferrique et l'oxyde ferrique hydraté, ou encore le dioxyde de titane (anatase, rutile ou amorphe), des oxydes de chrome, ou des sels complexes, le violet de manganèse, des bleus de Prusse, etc. Parmi les matières colorantes organiques de synthèse, on connaît les dérivés de cyanines, notamment les phtalocyanines telles que le Pigment Blue CI 77160, et les dérivés de quinoléine et d'anthraquinone, qui présentent généralement une bonne stabilité à la lumière et à l'oxydoréduction. On a également proposé des colorants comportant des groupements azo, nitroso et indigoïdes, ainsi que des triarylméthanes et des xanthènes, dont la stabilité vis-à-vis de l'oxydoréduction et du rayonnement UV est cependant médiocre.

Pour pouvoir être utilisées en cosmétique, ces matières colorantes doivent posséder de bonnes qualités de coloration et de stabilité, c'est-à-dire que les colorants doivent être non seulement efficaces et stables, mais ils doivent aussi procurer une coloration stable et compatible avec la fonction cosmétique ou dermatologique de la composition dans laquelle ils sont introduits.

La lumière et les agents oxydants et réducteurs sont les facteurs majeurs qui induisent une déstabilisation de la molécule colorante en provoquant une décomposition ou une réaction photochimique avec une autre molécule présente dans le milieu. Ainsi, le groupe azo présent dans de nombreux colorants peut être oxydé en groupe azoxy sous l'action de l'oxygène, pour former par exemple une azohydrazone, qui par dégradation aboutit à une naphtoquinone et une autre molécule colorante dérivée de sel d'aryldiazonium procurant une coloration différente de celle du colorant de départ. La lumière peut aussi induire des radicaux libres qui vont favoriser la réduction de la molécule colorante, même en présence d'oxygène. Cette dégradation des colorants sous l'action de composés réducteurs, par exemple des sucres, est souvent observée même en présence de filtres UV.

Les lécithines, ou phosphatidyl cholines, sont des phosphoglycérides généralement extraits de l'huile de soja. En raison de leurs propriétés rhéologiques, les lécithines sont souvent utilisées dans des émulsions pour conférer à la composition des propriétés de douceur et d'onctuosité.

Ainsi par exemple, le brevet FR 2.777.179 décrit une composition cosmétique pour application topique comprenant une lécithine de soja en association avec de l'eau et un liquide hydrophobe tel qu'un hydrocarbure saturé ou une cyclo-méthicone, dans des proportions déterminées pour améliorer l'homogénéité des formulations. Des émulsions à base d'esters d'acides gras, d'eau et de lécithine, pouvant contenir des principes actifs cosmétiques ou dermatologiques hydrophiles ou hydrophobes, sont décrites dans le brevet FR 2.177.182. Le brevet FR 2.466.273 décrit des émulsions à base de substances d'origine naturelle, préparées à froid en utilisant comme émulsionnant un mélange de lécithine et de saponine.

La demande WO 97.37637 décrit une composition à base de liposomes contenant une lécithine hydrogénée en combinaison avec un alcool (de préférence l'éthanol) destinée à faciliter la pénétration du principe actif (vitamine D) dans la peau. L'utilisation de lécithine en association avec des pigments à base de dioxyde de titane a aussi été décrite dans le brevet US 5.817.290 relatif à des compositions antisolaires sous forme d'émulsions préparées au moyen d'émulsionnants usuels tels que des sesquioléates, des esters éthoxylés, des esters de sorbitans, etc., et contenant des particules de dioxyde de titane enduites de lécithine afin de favoriser leur dispersion dans l'émulsion et d'améliorer ainsi leur capacité d'absorber les rayons UV.

Le brevet EP-A-312.343 décrit une composition capillaire colorante dans laquelle le colorant est associé à un tensioactif cationique sous forme cristallisée en phase lamellaire afin d'augmenter la rapidité de la coloration. Une autre composition pour coloration capillaire est décrite dans le brevet EP-A-706-788, selon lequel on mélange un colorant, un composé amphiphile non ionique et un tensioactif cationique. La demande WO-A-95.11000 concerne un stick pour les lèvres à base de cire, de gélifiant et d'émollient, contenant des pigments mais aucun colorant.

Cependant, toutes ces compositions ne répondent pas au problème de l'instabilité de nombreux colorants dans les émulsions utilisées en cosmétique et en dermatologie.

Il est donc souhaitable de pouvoir disposer d'une large gamme de colorants présentant de bonnes propriétés de stabilité, en particulier dans des compositions cosmétiques ou dermatologiques déterminées.

La présente invention a précisément pour objet une association d'au moins un colorant et d'au moins un émulsionnant, et plus particulièrement une nouvelle composition cosmétique et/ou dermatologique sous forme d'émulsion contenant au moins un colorant en combinaison avec au moins un émulsionnant, procurant une coloration stable.

La demanderesse a en effet découvert que l'on pouvait préparer des émulsions utilisables en cosmétique et en dermatologie, contenant des colorants procurant une coloration stable dans le temps et à la lumière, en utilisant des émulsionnants capables de former une émulsion de type lamellaire.

La composition cosmétique et/ou dermatologique conforme à la présente invention est sous forme d'émulsion et se distingue en ce qu'elle comprend, en combinaison, un colorant liposoluble ou lipodispersible physiologiquement acceptable et un émulsionnant capable de former des émulsions lamellaires stables constitué par une lécithine partiellement ou totalement hydrogénée, et un ou plusieurs sucres choisis parmi le glucose, le fructose, le saccharose, le maltose, le lactose, le glycogène, le ribose, le ribulose, le melibiose, l'érythrulose et le trehalose.

La présente invention a aussi pour objet un procédé de préparation de telles émulsions utilisables en cosmétique et/ou en dermatologie.

L'invention a encore pour objet l'utilisation d'un émulsionnant capable de former des émulsions lamellaires stables, pour stabiliser un ou plusieurs colorants liposolubles ou lipodispersibles, ou améliorer leur stabilité, dans une composition cosmétique ou dermatologique.

Les colorants utilisés dans l'invention peuvent être choisis parmi les colorants liposolubles ou lipodispersibles naturels ou synthétiques physiologiquement compatibles usuels dans les compositions cosmétiques et dermatologiques, et tout particulièrement les quinoléines, les anthraquinones, les triarylméthanes, les xanthènes, les cyanines, les phtalocyanines, les colorants nitroso, azo et indigoïdes.

Suivant une caractéristique préférentielle de la présente invention, la composition contient un émulsionnant en combinaison avec un ou plusieurs alcools gras.

L'alcool gras peut être choisi parmi les alcools gras naturels ou synthétiques à chaîne aliphatique linéaire ou ramifiée comportant 5 à 30 atomes de carbone, et de préférence 8 à 22 atomes de carbone, et par exemple les alcools caprylique, laurylique, myristylique, cétylique, cétéarylique, oléylique et stéarylique. Ces alcools gras peuvent être utilisés isolément ou en mélange.

Suivant une autre caractéristique préférentielle de l'invention, l'émulsion se trouve sous forme solide ou à viscosité élevée, et l'émulsionnant capable de former une émulsion lamellaire est une lécithine partiellement ou totalement hydrogénée.

L'expression "émulsion solide" utilisée ici possède sa signification usuelle dans la technique des produits utilisés en cosmétique et en dermatologie, c'est-à-dire qu'elle désigne des émulsions ayant la consistance, par exemple, des baumes ou des sticks pour les lèvres qui conservent leur forme sans qu'il soit nécessaire de les maintenir dans un conteneur. Les émulsions à viscosité élevée auxquelles peut s'appliquer l'invention peuvent être par exemple des masques ou des pommades, et d'une manière générale des émulsions dont la viscosité, mesurée dans des conditions normales, est supérieure à 100.000 cps environ.

La lécithine hydrogénée utilisée dans l'invention est de préférence une lécithine de soja hydrogénée ou une lécithine de tournesol hydrogénée. Les lécithines disponibles dans le commerce contiennent généralement de la lécithine pure (phosphatidyl choline) en mélange avec d'autres phosphoglycérides tels que céphaline (en particulier la phosphatidyl éthanol-amine) et le phosphatidyl inositol. Conformément à l'invention on peut utiliser la lécithine pure hydrogénée ou une lécithine du commerce hydrogénée, pourvu qu'elle permette de former une émulsion de type lamellaire. Comme exemple de lécithine hydrogénée utilisable dans l'invention on peut citer celles vendues dans le commerce sous les marques Emulmetik 320® et Biophilic H par la société Lucas Mayer (lécithine de soja hydrogénée). Comme indiqué ci-dessus, il est avantageux de combiner la lécithine à un alcool gras.

Les sucres utilisés dans les émulsions à usage cosmétique ou dermatologique suivant l'invention, présentent l'avantage de posséder des propriétés adoucissantes, cicatrisantes et antiradicalaires. Suivant une forme préférentielle de réalisation, les sucres sont incorporés sous forme de miel. Tous les miels peuvent être utilisés dans l'invention, et par exemple un miel d'acacia contenant un mélange de glucose, de fructose et de saccharose, ou un miel de sapin, de bruyère, de romarin, de trèfle, etc.

Suivant une variante conforme à la présente invention, l'émulsion peut contenir des dérivés vitaminiques hydrosolubles, et notamment des dérivés de la vitamine C tels qu'un ascorbylphosphate de sodium, un ascorbylsulfate, un glycosylascorbate, un sarcosinate d'ascorbyle, ou encore l'acide citrique ou un de ses sels et esters. Ces substances, présentes dans la phase aqueuse de l'émulsion, peuvent être utilisées en combinaison avec les sucres.

Les essais et expérimentations effectués ont montré qu'en combinant les colorants liposolubles ou lipodispersibles avec une émulsion lamellaire, on pouvait améliorer sensiblement leur stabilité, tant dans le temps qu'à la lumière, même en présence d'un composé réducteur tel qu'un sucre ou une vitamine telle que la vitamine C et ses dérivés. La double couche phospholipidique, combinée aux alcools gras présents dans la composition, permet d'emprisonner les molécules d'eau liées aux sucres, les têtes polaires se trouvant à l'extérieur et les longues chaînes apolaires à l'intérieur, pour former un réseau lamellaire en piégeant les sucres entre les têtes polaires.

Il devient ainsi possible, dans l'émulsion de l'invention, de protéger les colorants liposolubles ou lipodispersibles de l'action de composés qui, tels les sucres et autres composés réducteurs, pourraient les dégrader en provoquant une réaction d'oxydo-réduction. Ainsi, une émulsion conforme à la présente invention contenant des colorants et des pigments liposolubles ou lipodispersibles, mêmes ceux réputés instables comme le violet de manganèse et le Blue n°1 (dérivé du triarylméthane), peut aussi contenir des sucres tels que le glucose et le fructose, bien que ceux-ci puissent réduire les sels métalliques (pigments colorés) et la plupart des colorants organiques de synthèse.

Suivant une forme préférentielle de mise en oeuvre de l'invention, la composition comprend entre 50 et 75% en poids de matières grasses choisies parmi les alcools gras, les triglycérides et les huiles végétales, entre 0,5 et 3%, de préférence entre 0,8 et 2%, en poids de lécithine hydrogénée, entre 1 et 10% en poids d'un ou plusieurs sucres réducteurs (en solution aqueuse de 0,5 à 70%), et entre 0,1 et 10% en poids de colorants, le reste étant constitué par des adjuvants, excipients et supports usuels physiologiquement acceptables. Les alcools gras, qui sont inclus parmi les matières grasses, représentent généralement entre 0,5 et 25% en poids par rapport au poids total de l'émulsion.

Outre les colorants, émulsionnants et sucres mentionnés ci-dessus, la composition suivant la présente invention peut contenir divers adjuvants et excipients couramment utilisés dans les techniques des compositions cosmétiques et dermatologiques, et par exemple des conservateurs, des épaississants, des gélifiants hydrophiles ou lipophiles, des antioxydants, des agents hydratants, des tensioactifs, des parfums, et divers additifs destinés à améliorer les propriétés physiques de la composition. Il peut aussi être avantageux d'incorporer dans la composition de l'invention, des écrans ou filtres solaires actifs dans l'UVA et/ou l'UVB, choisis en fonction du degré de protection recherché.

Les gélifiants ou épaississants peuvent être choisis par exemple parmi les polyacrylamides (par exemple du type Carbopol), les copolymères acrylate/acide acrylique ou acrylamide/acide acrylamido propane sulfonique, les dérivés de cellulose comme l'hydroxypropyl cellulose, le chitosan, des mucilages, des muco-polysaccharides végétaux, les cires comme la cire d'abeille, ou les gommes naturelles comme la gomme de xanthane. Il est tout particulièrement avantageux d'utiliser un gélifiant lipophile, compatible avec une émulsion à face externe huileuse, et notamment une bentonite quaternaire lipophile, qui permet de stabiliser plus efficacement l'émulsion en fonction de la température et procure une meilleure viscosité sur une plage de température plus large.

L'agent hydratant ou humectant peut être choisi parmi un polyol, la glycérine (glycérol et des dérivés de glycérol), le sorbitol, le maltitol, le pentaérythritol, les polyacrylates et polyméthacrylates de glycéryle, les mucopolysaccharides tels que l'acide hyaluronique, des dérivés du chitosan et des dérivés de l'acide pyrrolidone carboxylique. D'une manière générale, tout agent hydratant convenant aux compositions cosmétiques ou dermatologiques peut être utilisé dans la présente invention. L'agent humectant est avantageusement introduit dans la phase aqueuse lors de la préparation de l'émulsion. La teneur en agent humectant est généralement comprise entre 0,1 et 10% en poids par rapport au poids total de l'émulsion.

On peut aussi ajouter des émollients tels qu'un malate d'alkyle, l'isohexadécane, le myristate d'isopropyle, des triglycérides d'acides gras tels que l'acide laurique, caprique ou caprylique, etc. On peut par exemple utiliser un émollient tel que le beurre de karité qui présente aussi des propriétés calmantes et adoucissantes. Une huile d'amandes douces peut être avantageusement incorporée dans l'émulsion pour ses propriétés émollientes, lubrifiantes et adoucissantes.

La composition de l'invention peut aussi comprendre d'autres émulsionnants compatibles, et par exemple, on peut y incorporer un émulsionnant additionnel choisi parmi ceux couramment employés dans la technique, à l'exception des émulsionnants polyéthoxylés. On peut utiliser par exemple un ester de sorbitan, tel que le stéarate ou le laurate de sorbitan, ou un dérivé de sucrose (par exemple un ester) non éthoxylé.

Il peut aussi être avantageux d'incorporer dans la composition un actif complémentaire destiné à améliorer le comportement de la peau, tel que le tocophérol, la vitamine A (rétinol), l'acide rétinoïque, des agents bactéricides, etc. Le tocophérol est tout particulièrement utile en raison de sa capacité à piéger les radicaux libres et ses propriétés d'anti-oxydant des corps gras.

Les compositions peuvent se présenter sous forme de crèmes, baumes, émulsions huile-dans-eau (H/E) ou émulsions eau-dans-huile (E/H), gels, masques, onguents ou pommades, et de préférence sous forme de baumes ou émulsions huile dans eau. Ces diverses formes galéniques sont préparées suivant les techniques usuelles. Ces compositions peuvent être présentées sous forme de produits de soins ou de maquillage, et par exemple de baume pour les lèvres.

La composition suivant l'invention est préparée par les techniques classiques, en fonction des composants utilisés. Par exemple, il est avantageux de procéder en plusieurs étapes consistant a) à préparer une phase grasse constituée par des corps gras, b) à incorporer un mélange d'huiles végétales, puis c) à ajouter ensuite une phase aqueuse contenant l'émulsionnant capable de former une émulsion lamellaire, et d) à ajouter enfin les colorants liposolubles ou lipodispersibles, et le cas échéant des huiles essentielles. Suivant une variante, on peut inverser les étapes b) et c), c'est-à-dire n'incorporer les huiles végétales qu'après avoir ajouté l'émulsionnant capable de former une émulsion lamellaire à la phase grasse préparées dans la première étape.

Dans la première étape a) ci-dessus, on peut mélanger par exemple des triglycérides, des huiles, un liant et un émollient, tandis que les sucres et les substances actives éventuellement présentes, telles que antiseptiques; cicatrisants, ainsi que les filtres UV, sont de préférence ajoutés avec les huiles végétales, dans les étapes b) ou c). Les alcools gras sont de préférence incorporés au cours de l'étape b) ou c).

Les tests de stabilité effectués en étuve et à la lumière du jour, ainsi que les mesures colorimétriques, ont montré que les compositions conformes à la présente invention présentent une excellente stabilité de coloration, résultant de l'efficacité du réseau lamellaire de l'émulsion, qui isole les molécules de colorants et empêche leur réduction par les sucres présents dans l'émulsion.

D'autres caractéristiques et avantages de la présente invention apparaîtront dans les exemples suivants, qui illustrent l'invention plus en détail sans en limiter la portée. Sauf indication contraire, les parties et pourcentages sont indiqués en poids.

### Exemple 1

On prépare une émulsion huile dans eau (H/E) destinée à un baume pour les lèvres en opérant comme indiqué ci-dessous, en utilisant les techniques usuelles de l'industrie des produits cosmétiques et dermatologiques.

La composition pondérale de l'émulsion est la suivante.

| Phase A : | |
|---|---|
| Beurre de karité | 8,00 g |
| Cire d'abeilles blanche | 2,00 g |
| Huiles végétales et cire de candelila (Cremeol VP de la société Saci CFPA) | 14,00 g |
| Diméthicone | 5,00 g |
| Allantoïne | 0,20 g |
| Di-isostéaryl malate | 13,90 g |

| Phase B : | |
|---|---|
| Huiles végétales | 17,00 g |
| Acétate de tocophérol | 0,10 g |
| Méthoxycinnamate d'octyle | 5,00 g |

| Phase C : | |
|---|---|
| Lécithine hydrogénée / alcools C₁₂-Cₗ₆ / acide palmitique (Biophilic H de Lucas Mayer) | 8,00 g |
| Miel d'acacia | 5,00 g |

| Phase D : | |
|---|---|
| Huiles essentielles de citron et pamplemousse | 3,00 g |
| Antioxydant | 0,70 g |
| Violet de manganèse (Mango Violet®) Wakherr | 8,50 g |
| Nacres (Cellini Red® - Mearl) | 9,60 g |

Les composants de la phase A sont chauffés au bain-marie à 80°C. La phase B est chauffée à 65°C jusqu'à homogénéisation puis elle est introduite dans la phase A. Les composants de la phase C sont chauffés au bain-marie jusqu'à une température d'environ 70°C et mélangés au moyen d'un agitateur à cage (Ultraturrax®) jusqu'à obtenir une masse homogène que l'on incorpore dans la phase précédente, sous agitation.

On obtient ainsi une émulsion lamellaire, que l'on maintient sous agitation à 55°C environ, puis on ajoute les composants de la phase D (sauf les colorants). On laisse refroidir à température ambiante et on ajoute alors les colorants et les nacres. Cette émulsion peut être utilisée comme baume pour les lèvres.

Le point de goutte de l'émulsion, mesuré avec un appareil Mettler Toledo® est de 77,3°C.

### Exemple 2

En procédant comme dans l'Exemple 1, on prépare une émulsion huile dans eau ayant la composition indiquée ci-après.

| Phase A : | |
|---|---|
| Cire de riz | 2,50 g |
| Allantoïne | 0,20 g |
| Cire d'abeilles blanche | 2,00 g |
| Cire de candelila | 3,00 g |
| Méthyl phényl polysiloxane | 5,00 g |
| Stéaryl triméthicone | 3,00 g |
| Di-isostéaryl malate | 9,00 g |
| Ricinoléate de cétyle | 2,30 g |

| Phase B : | |
|---|---|
| Beurre de Karité | 13,00 g |
| Huile d'amandes douces | 12,00 g |
| Acétate de tocophérol | 0,70 g |
| Méthoxycinnamate d'octyle | 5,00 g |

| Phase C1 : | |
|---|---|
| Glyceryl tribéhénate et calcium béhénate | 7,30 g |
| Bentonite quaternisée (Bentone Gel EUG) | 7,00 g |

| Phase C2 : | |
|---|---|
| Lécithine hydrogénée / alcools C₁₂-C₁₆ / acide palmitique (Biophilic H de Lucas Mayer) | 8,00 g |
| Glycogène | 1,00 g |
| Ascorbyl sulfate | 0,50 g |
| Methyl chitosan | 0,50 g |
| Eau | 3,00 g |
| Nylon 12 (microperles) | 6,00 g |

| Phase D : | |
|---|---|
| Huiles essentielles, parfum | 3,00 g |
| Violet de manganèse (Mango Violet®) Wakherr | 3,50 g |
| Oxyde de fer noir | 0,80 g |
| Oxyde de fer brun | 0,50 g |
| Red 7 / Red 28 laque d'aluminium | 1,20 g |

La préparation de l'émulsion se fait comme dans l'Exemple 1, mais les produits de la phase C2, c'est-à-dire la lécithine hydrogénée, l'alcool gras, l'eau et les produits hydrosolubles sont mélangés entre eux avant d'être ajoutés aux produits fondus et mélangés précédemment, afin d'encapsuler et d'isoler les substances réductrices.

### Exemple 3

Cet exemple montre l'influence de la lécithine hydrogénée de l'Exemple 1 sur la stabilité des colorants utilisés dans les compositions de l'invention.

On prépare une émulsion ayant la même composition que celle de l'Exemple 1 ci-dessus, mais en remplaçant la lécithine hydrogénée (Biophilic H®) par une lécithine de soja déshuilée (Emulmetik 300® de la société Lucas Mayer) et en opérant de la même manière, et en n'utilisant pas d'alcool gras.

On mesure le point de goutte de chaque émulsion, c'est-à-dire la température à laquelle elle se liquéfie, au moyen d'un appareil Mettler Toledo®, puis on effectue un test de stabilité et des mesures colorimétriques comme indiqué ci-après.

Les tests de stabilité sont réalisés en comparant chacune des émulsions de l'Exemple 1 et de l'Exemple 3 à un témoin placé à l'abri de la lumière, toutes conditions égales par ailleurs. Les mesures sont effectuées à la lumière du jour au bout de 7 jours, 28 jours, 2 mois et 3 mois respectivement, et une deuxième série de mesures est faite en étuve à 50°C, au bout des mêmes périodes.

Les mesures colorimétriques sont faites avec un appareil Minolta mesurant les paramètres suivants : clarté (L*), le rouge (+a*) jusqu'au vert (-a*), et le jaune (+b*) jusqu'au bleu (-b*) .

Les résultats sont regroupés dans les tableaux ci-après.

### Point de goutte

| | | |
|---|---|---|
| Point de goutte | Exemple 1 | Exemple 3 |
| | 77,3°C | 50°C |

### Test de stabilité

Les tests ont été effectués par rapport au témoin placé à l'abri de la lumière.

| Lumière du jour | 7 j | 28 j | 2 mois | 3 mois |
|---|---|---|---|---|
| Exemple 1 | Rose | Rose | Rose | Rose |
| Exemple 3 | décol. | Orange | jaune | jaune |

On constate que la couleur de l'émulsion conforme à l'Exemple 1 reste stable à la lumière du jour pendant au moins 3 mois tandis que l'émulsion de l'Exemple 3 présente une légère décoloration dès le 7^{ème} jour, et une coloration orange puis jaune au bout de 28 jours, puis 2 mois.

| Etuve 50°C | 7 j | 28 j | 2 mois | 3 mois |
|---|---|---|---|---|
| Exemple 1 | Rose | Rose | Rose | Rose |
| Exemple 3 | Décol. | + | ++ | +++ |

| | | | | |
|---|---|---|---|---|
| + : décoloration ++ : forte décoloration +++ : très forte décoloration. | | | | |

On constate que l'émulsion de l'Exemple 1, placée en étuve à 50°C, résiste à la décoloration, tandis que celle de l'Exemple 3 se décolore rapidement.

### Mesures colorimétriques

Le témoin est maintenu à l'abri de la lumière à température ambiante, tandis que l'émulsion de l'exemple est placée dans une étuve à 50°C.

| Emulsion de l'Exemple 1 | | | |
|---|---|---|---|
| | L* | a* | b* |
| Témoin 1 h | 62,15 | 22,03 | -1,52 |
| Ex 1. (3 mois) | 61,84 | 21,68 | -1,47 |

| Emulsion de l'Exemple 3 | | | |
|---|---|---|---|
| | L* | a* | b* |
| Témoin 1 h | 63,61 | 19,45 | 5,42 |
| Ex 3. (2 mois) | 65,42 | 15,19 | 26,04 |

Ces essais permettent de constater que les mesures colorimétriques sont supérieures dans le cas de l'émulsion de l'Exemple 1, conforme à l'invention, par comparaison avec celles de l'Exemple 3, bien que ces dernières soient effectuées 1 mois plus tôt. Ces mesures colorimétriques confirment la stabilité de l'émulsion suivant l'invention.

## Revendications

1. Composition cosmétique et/ou dermatologique sous forme d'émulsion stabilisée contenant au moins un émulsionnant et au moins un colorant liposoluble ou lipodispersible physiologiquement acceptable, **caractérisée en ce qu'**elle comprend un émulsionnant capable de former une émulsion lamellaire constitué par une lécithine partiellement ou totalement hydrogénée, et un ou plusieurs sucres choisis parmi le glucose, le fructose, le saccharose, le maltose, le lactose, le glycogène, le ribose, le ribulose, le melibiose, l'érythrulose et le trehalose.

2. Composition selon la revendication 1, **caractérisée en ce qu'**elle comprend un émulsionnant capable de former une émulsion lamellaire, en combinaison avec un alcool gras.

3. Composition selon la revendication 2, **caractérisée en ce que** l'alcool gras est choisi parmi les alcools caprylique, laurylique, myristylique, cétylique, cétéarylique, oléylique et stéarylique.

4. Composition selon l'une quelconque des revendications précédentes, **caractérisée en ce qu'**elle contient un ou plusieurs dérivés vitaminiques hydrosolubles.

5. Composition selon la revendication 4, **caractérisée en ce que** le dérivé vitaminique est choisi parmi un ascorbylphosphate de sodium, un ascorbylsulfate, un glycosylascorbate, un sarcosinate d'ascorbyle, et l'acide citrique ou un de ses sels et esters.

6. Composition selon la revendication 1, **caractérisée en ce que** le sucre est sous forme de miel, et de préférence un miel d'acacia, de sapin, de bruyère, de romarin, ou de trèfle.

7. Composition selon l'une quelconque des revendications 1 à 6, **caractérisée en ce qu'**elle comprend entre 50 et 75% en poids de matières grasses choisies parmi les alcools gras, les triglycérides et les huiles végétales, entre 0,5 et 3%, de préférence entre 0,8 et 2%, en poids de lécithine hydrogénée, entre 1 et 10% en poids d'un ou plusieurs sucres réducteurs (en solution aqueuse de 0,5 à 70%), et entre 0,1 et 10% en poids de colorants, ainsi que des adjuvants, excipients et supports usuels physiologiquement acceptables.

8. Composition selon la revendication 7, **caractérisée en ce que** la teneur en alcool gras est comprise entre 0,5 et 25% en poids par rapport au poids total de l'émulsion.

9. Utilisation d'au moins un émulsionnant capable de former des émulsions lamellaires constitué par une lécithine partiellement ou totalement hydrogénée, pour stabiliser ou améliorer la stabilité d'un colorant liposoluble ou lipodispersible dans une composition cosmétique ou dermatologique contenant un sucre.

10. Procédé de préparation d'une composition cosmétique selon la revendication 1, **caractérisé en ce qu'**il comporte les étapes consistant à a) préparer une phase grasse constituée par des corps gras, b) incorporer un mélange d'huiles végétales, puis c) ajouter ensuite une phase aqueuse contenant l'émulsionnant capable de former une émulsion lamellaire, et d) ajouter les colorants liposolubles ou lipodispersibles, et le cas échéant des huiles essentielles.

11. Procédé selon la revendication 10, **caractérisé en ce que** l'on inverse l'ordre des étapes b) et c).

12. Procédé selon l'une quelconque des revendications 10 et 11, **caractérisé en ce que** l'on réalise la première étape a) en mélangeant des triglycérides, des huiles, un liant et un émollient.

13. Procédé selon l'une quelconque des revendications 10 à 12, **caractérisé en ce qu'**un alcool gras est incorporé dans l'étape b) ou c).

14. Procédé selon l'une quelconque des revendications 10 à 13, **caractérisé en ce que** les sucres, des substances actives ainsi que des filtres UV, sont incorporés avec des huiles végétales dans l'étape b) ou l'étape c).

## Claims

1. Cosmetic and/or dermatological omposition in the form of a stabilised emulsion containing at least one emulsifier and at least one physiologically acceptable liposoluble or lipodispersible coloring agent, **characterized in that** it comprises an emulsifying agent capable of forming a lamellar emulsion, consisting of a lecithin partially or totally hydrogenated, and one or several sugars selected from glucose, fructose, saccharose, maltose, lactose, glycogen, ribose, ribulose, melibiose, erythrulose and trehalose.

2. Composition according to claim 1, **characterized in that** it comprises an emulsifying agent capable of forming a lamellar emulsion, in combination with a fatty alcohol.

3. Composition according to claim 2, **characterized in that** the fatty alcohol is selected from caprylic, laurylic, myristylic, cetylic, cetearylic, oleylic and stearylic alcohols.

4. Composition according to any one of the preceding claims, **characterized in that** it contains one or several watersoluble vitamin derivatives.

5. Composition according to claim 4, **characterized in that** the vitamin derivative is selected from sodium ascorbylphosphate, ascorbylsulfate, glycosylascorbate, ascorbyl sarcosinate, and citric acid or one of its salts or esters.

6. Composition according to claim 1, **characterized in that** the sugar is in the form of honey, and preferably acacia, fir, heather, rosemary, or clover honey.

7. Composition according to any one of claims 1 to 6, **characterized in that** it comprises from 50 to 75% by weight of fatty materials selected from fatty alcohols, triglycerides and vegetal oils, from 0, 5 to 3%, preferably from 0,8 to 2%, by weight of hydrogenated lecithin, from 1 to 10% by weight of one or several reducing sugars (in aqueous solution from 0,5 to 70%), and from 0,1 to 10% by weight of coloring agent, as well as usual physiologically acceptable additives, excipients and supports.

8. Composition according to claim 7, **characterized in that** the content of fatty alcohol is comprised between 0,5 and 25% by weight of the total weight of the emulsion.

9. Use of at least one emulsifying agent capable of forming lamellar emulsions, consisting of partially or totally hydrogenated lecithin, in order to stabilise or improve the stability of a liposoluble or lipodispersible coloring agent in a cosmetic or dermatological composition containing a sugar.

10. Process for the preparation of a cosmetic composition according to claim 1, **characterized in that** it comprises the steps consisting in a) preparing a fatty phase consisting of fatty materials, b) incorporating a vegetal oils mixture, then c) adding an aqueous phase containing the emulsifying agent capable of forming a lamellar emulsion, and d) adding the liposoluble or lipodispersible coloring agents, and if necessary essential oils.

11. Process according to claim 10, **characterized in that** the order of steps b) and c) is reversed.

12. Process according to any one of claims 10 and 11, **characterized in that** the first step a) is carried out by mixing triglycerides, oils, a binding agent and a emollient.

13. Process according to any one of claims 10 to 12, **characterized in that** a fatty alcohol is incorporated in step b) or c).

14. Process according to any one of claims 10 to 13, **characterized in that** sugars, active substances and UV filters, are incorporated with vegetal oils in step b) or step C) .

## Patentansprüche

1. Kosmetische und/oder dermatologische Zusammensetzung in Form einer stabilisierten Emulsion, die zumindest einen Emulgator und zumindest einen physiologisch annehmbaren, in Fett löslichen oder dispergierbaren Farbstoff enthält, **dadurch gekennzeichnet, dass** sie einen Emulgator umfasst, der in der Lage ist, eine lamellare Emulsion zu bilden, die aus einem partiell oder vollständig hydrierten Lecithin und einem oder mehreren aus Glucose, Fructose, Saccharose, Maltose, Lactose, Glykogen, Ribose, Ribulose, Melibiose, Erythrulose und Trehalose ausgewählten Zuckern besteht.

2. Zusammensetzung nach Anspruch 1, **dadurch gekennzeichnet, dass** sie einen Emulgator, der in der Lage ist, eine lamellare Emulsion zu bilden, in Kombination mit einem Fettalkohol umfasst.

3. Zusammensetzung nach Anspruch 2, **dadurch gekennzeichnet, dass** der Fettalkohol aus Capryl-, Lauryl-, Myristyl-, Cetyl-, Cetearyl-, Oleyl- und Stearylalkohol ausgewählt ist.

4. Zusammensetzung nach einem der vorangegangenen Ansprüche, **dadurch gekennzeichnet, dass** sie ein oder mehrere wasserlösliche Vitaminderivate enthält.

5. Zusammensetzung nach Anspruch 4, **dadurch gekennzeichnet, dass** das Vitaminderivat aus Natriumascorbylphosphat, Ascorbylsulfat, Glykosylascorbat, Ascorbylsarcosinat, Zitronensäure oder eines Salzes oder Esters davon ausgewählt ist.

6. Zusammensetzung nach Anspruch 1, **dadurch gekennzeichnet, dass** der Zucker in Form von Honig, vorzugsweise in Form von Akazien-, Tannen-, Heidekraut-, Rosmarin- oder Kleehonig, vorliegt.

7. Zusammensetzung nach einem der Ansprüche 1 bis 6, **dadurch gekennzeichnet, dass** sie Folgendes umfasst: zwischen 50 und 75 Gew.-% Fettstoffen, die aus Fettalkoholen, Triglyceriden und pflanzlichen Ölen ausgewählt sind; zwischen 0,5 und 3 Gew.-%, vorzugsweise zwischen 0,8 und 2 Gew.-%, hydriertes Lecithin; zwischen 1 und 10 Gew.-% eines oder mehrerer reduzierender Zucker (in 0,5- bis 70%iger wässriger Lösung) und zwischen 0,1 und 10 Gew.-% Farbstoffe, sowie physiologisch annehmbare, herkömmliche Adjuvanzien, Exzipienten und Träger.

8. Zusammensetzung nach Anspruch 7, **dadurch gekennzeichnet, dass** der Fettalkoholgehalt zwischen 0,5 und 25 Gew.-%, bezogen auf das Gesamtgewicht der Emulsion, liegt.

9. Verwendung zumindest eines Emulgators, der in der Lage ist, lamellare Emulsionen zu bilden und aus einem partiell oder vollständig hydrierten Lecithin besteht, zur Stabilisierung oder Verbesserung der Stabilität eines in Fett löslichen oder dispergierbaren Farbstoffs einer kosmetischen oder dermatologischen Zusammensetzung, die einen Zucker enthält.

10. Verfahren zur Herstellung einer kosmetischen Zusammensetzung nach Anspruch 1, **dadurch gekennzeichnet, dass** es folgende Schritte umfasst: a) die Herstellung einer aus Fettstoffen gebildeten Fettphase, b) das Einmischen eines Gemischs aus Pflanzenölen und anschließend c) das Zusetzen einer wässrigen Phase, die den Emulgator enthält, der in der Lage ist, eine lamellare Emulsion zu bilden, und d) das Zusetzen der in Fett löslichen oder dispergierbaren Farbstoffe sowie gegebenenfalls von ätherischen Ölen.

11. Verfahren nach Anspruch 10, **dadurch gekennzeichnet, dass** die Reihenfolge der Schritte b) und c) umgekehrt wird.

12. Verfahren nach einem der Ansprüche 10 und 11, **dadurch gekennzeichnet, dass** der erste Schritt a) durchgeführt wird, indem Triglyceride, Öle, ein Bindemittel und ein Weichmacher vermischt werden.

13. Verfahren nach einem der Ansprüche 10 bis 12, **dadurch gekennzeichnet, dass** in Schritt b) oder c) ein Fettalkohol eingemischt wird.

14. Verfahren nach einem der Ansprüche 10 bis 13, **dadurch gekennzeichnet, dass** die Zucker, aktive Substanzen, wie z.B. UV-Filter, in Schritt b) oder c) gemeinsam mit den Pflanzenölen eingemischt werden.
